## Europäisches Patentamt

## European Patent Office

## Office européen des brevets

(11) Publication number: **0 066 283**
**B1**

## (12) EUROPEAN PATENT SPECIFICATION

(45) Date of publication of patent specification: **10.09.86**

(51) Int. Cl.⁴: **A 61 K 31/725, A 61 K 45/06**

(21) Application number: **82104734.7**

(22) Date of filing: **28.05.82**

(54) Eustatic composition for nonspecifically facilitating and amplifying the generalized homeostatic regulation and maintenance, compensation and repair in living organisms.

(30) Priority: **02.06.81 US 269717**

(43) Date of publication of application:
**08.12.82 Bulletin 82/49**

(45) Publication of the grant of the patent:
**10.09.86 Bulletin 86/37**

(84) Designated Contracting States:
**DE FR GB IT**

(56) References cited:
**EP-A-0 000 133**
**EP-A-0 012 115**
**FR-A-1 176 917**
**US-A-4 058 621**

(73) Proprietor: **EUPAN CORPORATION**
**320 East 58th Street**
**New York, N.Y. 10022 (US)**

(72) Inventor: **Osmundsen, John A.**
**320 East 58th Street**
**New York, N. Y. 10022 (US)**

(74) Representative: **Wuesthoff, Franz, Dr.-Ing. et al**
**Patentanwälte Wuesthoff -v. Pechmann-**
**Behrens-Goetz Schweigerstrasse 2**
**D-8000 München 90 (DE)**

**Description**

This invention relates to agents, for nonspecifically facilitating and enhancing the normal homeostatic regulation and repair processes in living organisms. These agents are called "eustatants".

In living organisms, there is constant pressure from regulatory mechanisms toward metabolic stabilization, or health, and away from the pathology of injury, or disease. Thus, if injury is defined in its most inclusive sense as any forcible unbalancing of the steady state of an organism, not all injury will result in disease or ill-health. Undetected injuries are constantly occurring and being corrected by the organism's internal system of metabolic regulation and preservation. This concept of internal metabolic regulation has been termed "homeostasis."

Homeostasis embraces the full range of physiological responses to almost all conceivable injuries, and also accounts for the continuous regulation of all routine maintenance phenomena, such as amino acid synthesis, blood pressure, neuromuscular control, respiration, excretion and body temperature. What we call herein the "Homeostatic Regulatory System," or HRS, is an organism's inherent system of defense against disease-producing injury and its capacity for maintaining vital life functions. The HRS involves all the cells of an organism.

Obviously, there are limits to the capacity of the HRS to perform its function of maintaining the integrity of the living organism, i.e., the injury to the organism may be so extensive that the HRS is overwhelmed at least temporarily. In that event, specific external agents — usually in the form of medications — are required in order to restore the organism to health. An example of such a medication is provided by prior art European Patent Publication No. 133 of Ciba-Geigy AG, which discloses a pharmaceutical preparation for topical administration for the treatment of virus infections. The preparation contains a combination of an acid sulphated polysaccharide or polymer, heparin in particular, and zinc ions, and is useful especially in the treatment of infections caused by herpes viruses.

To minimize the occurrence of traumas which overwhelm the HRS, it would be of great benefit if completely innocuous means were found for enhancing an organism's ordinary homeostatic ability to maintain itself and counteract the injuries of disease and degeneration. Such means would produce a general, pervasive augmentation of normal HRS activity while, at the same time, enhancing the organism's specific capacity to respond to specific injuries.

Despite the immense benefit that might accrue from devising ways of promoting HRS activity, the immense complexity of the system seems up to now to have discouraged notions of finding a general stimulant to homeostasis. One fact that is clear, however, is that the underlying means by which homeostatic regulation is modulated is the process of information transfer among the cells of the organism. As a result of advancement in the past ten to fifteen years in the understanding of the mechanism by which the cells of an organism "communicate" with the one another, it has now become possible to devise a variety of external agents or agencies for producing a general rise in HRS activity with all the attendant consequences of enhanced — and appropriate — defense, repair and maintenance effects.

Such exogenous agents or agencies are herein termed "eustatants," the prefix "eu" indicating a betterment of ordinary homeostasis. "Eustasis" can be defined simply as the catalytic amplification of normal cellular communications that are involved in homeostatic regulatory processess. In order to understand how "eustatants" have been identified and how the administration of such substances can generally facilitate and amplify the process of information transfer among cells, a brief summary of current knowledge about intercellular communications is helpful.

Intercellular communication in higher animals takes place in essentially two ways. One way involves· the nervous system; the other involves the endocrine system of secretory cells. Information transfer in the nervous system occurs directly from one call to another across intercellular junctions called synapses via chemical agents called neurotransmitters. Information transfer via the endocrine system occurs indirectly, through hormones that are disseminated throughout the organism by the circulatory systems.

Both neurotransmitters and endocrine hormones, together often called "mediators", interact with specialized sites called "receptors", on the surfaces of cells being communicated with. Mediators originate in a variety of ways, from the stimulation of certain cells by various factors in the external environment (e.g., irradiation, toxins), to metabolic processes occurring within the organism.

In the first step of the intercellular communications process, any specific mediator-receptor engagement at the cell surface provokes the activation of adenylate cyclase within the cell membrane. In the next step, adenylate cyclase degrades molecules of the chief energy-storage substance of cells, called adenosine triphosphate, or ATP. The removal of two of its three high-energy phosphate groups converts ATP into adenosine monophosphate, or more precisely, cyclic AMP or "cAMP". The action of cAMP on preformed but inactive enzymes in the cell, called kinases, allows those enzymes then to go forth and activate still other preformed but inactive enzymes and hormones in the cell whose particular functions correspond appropriately to the needs of the cell for survival and of the organism for maintenance. Those particular needs are reflected at any given moment by the messages received by the cell in the form of mediators and by the prevailing electrochemical microenvironment of the cell (and organism). Different cell types are distinguished not only by their surfaces, but by the enzymes and hormones they contain and which determine the metabolic functions that are characteristic of those cells. Thus, only certain kinds of kinase-activatable enzymes and hormones are present in cells of a particular type, and the universal cAMP will therefore cause only those specific molecules to be activated.

The kinase-activatable enzymes and hormones present in the cell are pre-formed by the cell's genome — the genes contained in the rod-like chromosomes that reside in the cell nucleus and carry the programmed genetic information that confers its biochemical (structural, compositional, functional) identity upon each cell and the parent organism. Other enzymes and hormones are produced by direct stimulation of the genome. For example, steroid hormones in most instances move directly to the genome rather than, as the peptide hormones do, triggering adenylate cyclase activity in the cell's outer (plasma) membrane. Coupled with its glycoprotein receptor site, steroid hormone travels through channels in the cell's cytocavitary network of intracellular membranes. When it arrives at the nucleus, the hormone-receptor complex induces activity of a particular gene whose function it is to promote the production of a certain protein — e.g., an enzyme or other hormone — which represents a specific response to the mediator's hormonal message.

Thus, cells generate protein responses (in the form of enzymes and hormones) to mediator-borne messages from outside the cell in essentially two ways, both of which are tied to the genome. One way is via the kind of mediator-receptor interaction that stimulates adenylate cyclase-cAMP activation of enzymes and hormones that were produced earlier in inactive form by the genome. This mode of protein generation by activation is much faster than the other route, in which the steroid hormone-receptor site travels to the genome for direct, specific induction of genetic activity. Thus, a differential in rate of response to messages of different sorts represents yet another modulatory control on homeostatic processes.

Hormone mediators need not always be involved in communications with the genome. Chemical constituents of the intracellular fluid (cytoplasm), or even from the intercellular spaces outside the cell, can induce the genome to promote the synthesis of molecules appropriate to the circumstances represented by the stimulating agent.

It should be clear that the essential elements of the cellular communications system are the genome and cell membranes. The latter includes the cell's surrounding plasma membranes and associated cytocavitary network of membranes inside the cell. The mechanisms by which the genome and cell membranes mediate information-transfer processes in cells reveal much about the basic mechanisms of eustasis itself, and how eustatants can be employed to amplify homeostasis processes in health and disease.

The genome

The genome performs two operations on the information it carries encoded in the structure of the deoxyribonucleic acid (DNA) of which its component genes are made. One function is transmission of genetic information. This is accomplished by replication of both strands of the double-helical DNA during cell division (reproduction); one copy of the genome goes to each of the new cells. The other operation — transcription of genetic information — is the one that is performed during homeostatic regulatory activity. Transcription consists of the transfer of information encoded in the structure of DNA to the structure of a messenger, ribonucleic acid (mRNA). Genetic information now encoded in the structure of the mRNA is finally translated into the assembly of sequences of amino acids that constitute the proteins (e.g., enzymes, hormones) that are needed by the cell for various life-sustaining purposes.

The human genome is known to contain between 30,000 and 100,000 genes whose structures — informational contents — are spelled out in the sequences of part of the six billion nucleotide pairs that constitute human DNA. Stretched out in a long, double-helical strand, that much DNA would measure two meters (more than six feet) in length. Not only must all of that chemically coded information be contained within a volume of less than ten one-millionths of a meter in diameter — the cell nucleus — but the DNA must be able there both to replicate itself with a high degree of fidelity and to transcribe the information it bears into the structure of messenger RNA. The difficult feat of packing so much information into so small a space is made possible by a trebled coiling of the genetic material on itself. First, a stretch of DNA double helix is wound around little balls of histone protein at intervals of every 200 nucleotides, with about 140 nucleotides wrapping each histone ball. That "string of beads" is then coiled into another coil with about six of the beads in every turn. Finally, that coiled coil is looped once more on itself and attached to a protein scaffold — the entire assembly constituting a chromosome, one of the 46 in the human cell nucleus.

In actuality, only about 1/200th of the DNA of the genome, or roughly 30 million nucleotide pairs out of the six billion in the entire genome, actually code for protein synthesis. Presumably, the genetically inert regions of the DNA function at least partly to permit the intricate packing described above. According to this view, genetically active regions are located where mediators and other inducing substances can reach them most easily. Presumably, also, there is some priority to the arrangement of even those active regions, the most vital ones securing the most accessible positions, with regions of lesser value to the cell's (organism's) maintenance and survival occupying positions that either are more difficult to reach or are least unlikely to become occluded by injury or accident in the course of repeated replication of the genome.

In the context of eustasis, then, agents or agencies which would facilitate intercellular communications via action on the genome would necessarily do so by enhancing the transcription of genetic information from DNA to mRNA. This might be done by producing a reversible "relaxation" of some sort in the genome so that its constituent genes would become more easily assessible for transcription.

3

Cell membranes

According to the "fluid mosaic model," the plasma membrane which surrounds the cell can be thought of as a sandwich of three layers. The top layer makes contact with the cell's external environment through the substance of the intercellular space outside. The bottom layer forms an interface with the cell's cytoplasm and cytocavitary network inside. Each outer layer is composed of electrically charged polar head groups of phospholipid molecules. The intermediate layer consists of non-polar fatty acid chains of the phospholipids.

Also inside the sandwich are scattered lumps of globular proteins that sometimes protrude through one or both outer layers. In addition, glycosides — complex carbohydrates — are attached to some of the lipids (as glycolipids) and to some of the proteins (as glycoproteins). The glycosides crop out of both surfaces, on the exterior and the interior, of the cell's plasma membrane. Those protuberances of protein mark regions of the cell's receptor sites. The resultant structure is, thus, a mosaic of essentially globular proteins (and glycoside complexes) in a viscous fluid matrix of lipid (fatty substance).

That structure serves many purposes. For example, cell membranes can act as pumps for actively transporting certain substances in and out of the cell or various cell structures. They also participate in the charge-transfer interactions among species of molecules and ions in the cellular environment. The cell membrane also serves as a supporting matrix for enzymes (e.g., adenylate cyclase) and their actions. Its plasticity enables the plasmalemma to engulf substances in the surrounding milieu and imbibe them for use inside the cell and also to pass internal packets of cell-produced substances for excretion into the intercellular space.

All those functions and others are intrinsically involved in cellular communications and, so, are essential contributors to homeostatic regulation. A central aspect of the present invention, then, is the discovery how membrane and genomic function might be enhanced to improve cellular communications.

It is an object of the present invention to provide an agent for facilitating and amplifying the normal activity of the Homeostatic Regulatory System (HRS) of an organism, thereby promoting the optimal functioning thereof.

It is another object of the present invention to provide an agent for facilitating and amplifying the normal activity of the HRS of a living organism which does not produce deleterious side effects.

It is yet another object of the present invention to provide an agent for promoting the generalized optimal physiological functioning of an organism within its normal range while simultaneously enhancing the organism's specific responses to specific injuries.

It is a further object of the present invention to identify a new class of pharmacologic, nutritional, therapeutic and tonic agents, herein called "eustatants".

It is a still further object of the present invention to provide formulations of eustatants which can be administered to humans and other living organisms.

These and other objects of the invention will be apparent from the description and the examples hereinafter set forth.

The invention

The eustatic agents of the present invention accomplish the aforementioned objects by augmenting aspects of the normal activity of the basic cellular components of the intercellular communications system — the genome and the cell membranes. In this connection several circumstantial factors influencing the activity of both genome and cell membranes can be identified as being involved in the enhancement of cellular communications, including:

1 Accessability to cell membranes by mediators and other "informational substances";
2. Availability of specificity mechanisms, such as unobstructed receptor sites and matching mediator-receptor encounters;
3. Penetrability of cells by substances moving through the plasma membrane;
4. Conduction of informational substances to appropriate cellular organelles, including the nucleus;
5. Expressability of the genome as determined partly by the exposure of genes to informational substances.

In operational terms, those communications-related factors suggest the following means of functional facilitation of each one, respectively:

1. Facilitated chemical interaction among cells by either increased flow-rate of chemical species or an increased permeability of the intercellular substance through which mediators and other components of the cells' microenvironment must pass to reach cell membranes, or both;
2. Open receptor sites and increases probability of encounters with matching mediators and, perhaps, increased rate of receptor site replacement;
3. Increased plasma membrane activity, especially permeability and pinocytosis;
4. Increased electrochemical activity of the cytocavitary network, including the nuclear envelope;
5. A relaxation of some sort in the genome, perhaps by a change in the nuclear membrane's barrier potential or a reversible unwinding of the DNA, or both, for greater interactions between the genome and mediators and other cytoplasmic constituants.

Achieving any one of the above effects on the intercellular and intracellular environment should produce signs of at least mild enhancement, or amplification, of homeostatic regulation, or eustasis. Achieving several or all of them should produce marked or even extraordinary eustatic consequences.

Agents and agencies have been discovered which may promote these effects. These agents and agencies and the suggested mechanism by which they work are described in detail below.

Pseudoaqueous substances

The category of pseudoaqueous substances encompasses dimethyl sulfoxide (DMSO) and other compounds having a strong solvent action through living tissue which have electrochemical properties, such as dielectric constant and charge transfer capability, closely resembling those of water. Other compounds in this group would include the toxic chemical dimethyl formamide (DMF) and non-toxic ethylene cyclic carbonate and N-methyl-2-pyrrolidinone.

In the context of cellular communications, the powerful solvent action of these compounds serves to flush nutrients, ions and various informational substances throughout the organism to and often through cell membranes, even to the genome. They may also affect membrane function, modifying the electrochemical microenvironment of those structures by facilitating the rate and degree of exposure of membranes to both charged and uncharged chemical species from throughout the organism, and altering the composition of the membrane-bound chemical population. Pseudoaqueous eustatants may also act by clearing blocked receptor sites and facilitating mediator-receptor match-ups.

Metal ions

Certain metal ions, preferably divalent metal cations, have a selective affinity for reiterative nucleotide sequences in the DNA molecule and, when bound there, cause a reversible unwinding of the double helix. This action may be expected to increase the exposure of the genome to constituents of its immediate cellular environment and, in that way, presumably facilitate optimal genetic expression. In addition, divalent metal cations are known to bind strongly to cell membranes, thereby altering their structure and function. Also, the mere presence of these in the vicinity of membranes will alter their electrochemical microenvironment, influencing membrane function in that way as well.

An additional role for divalent cations to play in eustatic (and homeostatic) processes, which is hereinafter discussed in greater detail, involves the requirement for them in the depolymerization of hyaluronic acid in interstitial regions by reducing agents, an effect that increases access to cell surfaces.

Ions that have been chiefly associated with these effects and which are apparently tolerated by cells (at least in small concentrations) without significant irreversible toxicity include zinc, manganese, iron (the ferrous ion), copper (the cupric ion) and trivalent chromium (the latter two in much lower concentrations than the former because of their greater toxicity).

Glycosaminoglycans

One of the most common and visible of all corrective, i.e., homeostatic, responses to injury is the inflammatory process. The acute inflammatory response is highly complex but can be reduced essentially to two independent events.

Both represent aspects of cellular communications. One event is the accumulation of scavenger cells — leukocytes — that are summoned to the site of injury by chemical messages sent out by the injured cells. The other event consists of an alteration in the permeability of cells and tissues. This creates freer movement by leukocytes and other corrective substances — e.g., antibodies — into the injured region, there to perform their defensive/reparative functions. Clearly, the inducement of the modified permeability of cells and tissues that occurs as part of the inflammatory process without also inducing the other aspects of the inflammatory process — e.g., the accummulation of leukocytes — would have eustatic consequences, i.e., an amplifying effect on intercellular communications.

The suggestion that such a result would be achieved in an organism by the administration of glycosaminoglycans has been garnered from published reports of experiments performed in the 1950's. Given the ubiquity of the classic homeostatic process of wound healing and the suggestion that glycosaminoglycans play a role in it, it has been possible to identify one glycosaminoglycan of particular importance—hyaluronic acid. Not only is hyaluronic acid present in appreciable quantities in all cartilage and in more other tissues than any other glycosaminoglycan, it is an important constituent of the pervasive intercellular matrix. The following known facts are pertinent to the participation of hyaluronic acid in the facilitation of cellular communications.

1. Like other glycosaminoglycans, hyaluronic acid exists naturally as a polymer, a long chain of repeating disaccharide units (a glucuronic acid and a glucosamine) linked covalently (strongly) to a polypeptide (protein-like) backbone as a "proteoglycan" — a class of substances that forms the ground substance in the extracellular matrix of connective tissue.
2. The hyaluronic acid polymer carries a net negative electrical charge and, thus is classed as an anion, or more precisely, a polyanion.
3. In its native state as a sticky gel between cells, the hyaluronic acid portion of the proteoglycan matrix is a relatively large polymer with a molecular weight of around 10 million — too large, as such, to enter cells.

5

4. The hyaluronic acid component of intercellular proteoglycans depolymerizes into lower molecular-weight fragments (that are small enough to enter cells) by the action of enzymes of two kinds — certain glycosidases and proteases that degrade the carbohydrate and polypeptide parts of the proteoglycan, respectively.

5. Proteoglycans are large polyvalent anions which so tightly bind cations, such as metal ions, that they appear uncharged. Proteoglycans tend to aggregate, and this tendency is enhanced by polyvalent cations such as $Ca^{2+}$. Larger chains, such as that of hyaluronic acid, coil in a relatively random way and therefore may occupy a volume largely filled with solvent water to which small molecules or ions have access, but from which larger molecules may be excluded. This volume is the domain of the polysaccharide. Degraded glycosaminoglycans are excreted in the urine, their observed size suggesting that they have been partially degraded by glycosidases. Human adults degrade about 250 milligrams of proteoglycans per day, but since normal urine contains only a few milligrams of the partially degraded glycosaminoglycans, considerable degradation must occur in the tissues.

6. Hyaluronic acid is constantly being formed in tissues by the action of enzymes called transferases. It is depolymerized both enzymatically, by certain glycosidases (especially hyaluronidase) and non-enzymatically by oxidation-reduction systems and by ionizing radiation.

From the foregoing facts, the mechanisms by which hyaluronic acid, the cement substance in the intercellular matrix contributes to its own degradation, producing the necessary loosening of the junctional zones for the percolation of substances to cell surfaces, become evident. In addition, a means by which hyaluronic acid can play a role in the function of the genome is suggested, namely by carrying into cells DNA-unwinding metal cations. Thus, this glycosaminoglycan undoubtedly performs not just one but at least two key functions in the facilitation of cellular communications during homeostatic (and eustatic) regulatory activity.

The following scenario describes the way hyaluronic acid acts as a native homeostatant or an administered eustatant.

Low molecular-weight fragments of hyaluronic acid — either the native stuff degraded naturally in tissues or administered in the form of powdered cartilage (or some other tissue source) — assemble into separate small domains or zones of a gel. These hyaluronic gel-cells may contain nutrients, small informational substances, water and positively charged metal ions. The metal ions, as indicated earlier, can act as homeostatants or eustatants, themselves. If the ordinarily negatively-charged hyaluronic acid gel-cell has bound to a positively charged metal ion, the combination is electrically neutralized and may be small enough to enter a cell, presumably through an engulfment process as pinocytosis.

The presence of hyaluronic acid inside the cell can then induce the genome to promote the synthesis of hyauluronidase, the hydrolytic enzyme whose specific substrate is hyaluronic acid. That enzyme would then depolymerize the gel-cell, causing release of its contents of water, nutrients and metal ions. The metal ions would bind to DNA and produce a reversible unwinding of the genome, exposing it more fully to whatever informational substances were either already present in the cytoplasm or carried in by the hyaluronic acid gel-cell.

Both the hyaluronidase, synthesized under the direction of the genome in response to the presence of hyaluronic acid, and the fragments of gel-cells that the enzyme has degraded may then be excreted from the cell into the extracellular space. There the enzyme can continue its depolymerization of hyaluronic acid in the cement, producing a further loosening of the matrix for enhanced percolation of substances to cell surfaces. Some of those substances may be gel-cells newly generated by the action of the enzyme. And, of course, the excreted fragments of gel-cells can reaggregate as hyaluronic acid — along with the polymer synthesized by transferases — in the proteoglycan matrix, restoring the cement for the process to begin again.

It should be clear that the above process is cyclic, an autocatalytic amplification effect. Thus, one substance (hyaluronic acid) catalyzes the production of another (hyaluronidase) that catalyzes the generation of a form of the first substance (the gel-cell) that can re-enter the cycle, amplifying the overall reaction sequence.

This process undoubtedly occurs at a very low rate constantly in organisms, contributing minutely but continuously to homeostatic regulatory processes that maintain the organism's statistical edge against perpetual injury and imbalances in maintenance requirements for health and survival. Exogenous administration of hyaluronic acid will increase the rate of the process, thus producing the greater permeability of the intercellular substance. In addition, the hyaluronic gel-cells serve to carry metal ions to the genome which bind to the DNA and produce the reversible unwinding of the genome previously discussed.

The preferred glycosaminoglycan for administration as a eustatant is hyaluronic acid, which, can be obtained in quite pure form from the vitreous body of the eye and from the placenta and umbilical cord. Alternatives include mixtures of hyaluronic acid and the sulfated chondroitins, i.e., chondroitin sulfates A and C, which may be derived from deproteinated cartilage, and mixtures of the hyaluronic acid amino sugar and sugar acid glycosidic components — N-acetyl D-glucosamine and D-glucuronic acid, respectively (which mixtures shall be included in the term "glycosaminoglycan" for purposes of this application) — which may be derived from deproteinated chitin.

## 0 066 283

Non-toxic saponin glycosides

Glycoside synthesis goes on in all living cells, plant and animal alike. Glycosides are derived from simple sugars — monosaccharides — from which each acquires its generic name. Thus, a glycoside that is derived from the monosaccharide glucose is a glucoside, one from galactose a galactoside, from fructose a fructoside, etc. Moreover, many biologically important glycosides consist of modified monosaccharides; these include the sugar acids, amino sugars and deoxysugars. For example, modification of glucose by oxidation produces the important sugar acid glucuronic acid that plays a key role in many detoxification reactions. Another well-known sugar acid is L-xyloascorbic acid, or vitamin C. Among the amino sugars, the most important and widespread are the glucosamines, galactosamines and sialic acids, particularly neuraminic acid. And, of course, the deoxysugar deoxyribose is a key structural constituent of the genetic material, deoxyribonucleic acid.

Many glycosides contain non-carbohydrate components, most often alcohols, phenols and sterols. Examples of these are to be found among spices, vegetable dyes and drugs, including the well-known "cardiac glycosides", digitoxin and ouabain, both steroid-containing saponin glycosides. Finally, glycosides rarely occur by themselves in nature, but are linked together as disaccharides (e.g., maltose, consisting of two glucoses; lactose, a glucose and a galactose, sucrose, a glucose and a fructose) and as small sugar polymers, called oligosaccharides, and larger sugar polymers, called polysaccharides. Nearly all glycosides in higher organisms are attached to proteins and lipids as glycoproteins (and proteoglycans) and glycolipids, respectively, both of which abound on the surface of cell membranes.

The single property of glycosides that, more than any other, both determines and mediates their role in intercellular communications is their selective affinity for one another. The chief site for this sort of glycosidic activity is the cell surface.

The outer surface of the cell membrane can be thought of as a smooth lawn of phospholipids with a lot of glycosidic "weeds" growing out of it. These glycoproteins and glycolipids form what is called a "glycocalyx" over the cell surface, which may sometimes actually interfere with mediator-receptor site interactions. In its positive role, however, the qualitative composition of the glycosidic components of the glycocalyx is thought to give each cell type its characteristic identity.

The patterns of glycosides on cell surfaces serve as binding sites for a variety of substances, particularly other glycosides. For example, cardiac glycosides — the saponins digitoxin, ouabain and phlorhizin — bind tightly to the glycocalyx of the cardiac cell and interfere with ion and energy transfer across its membrane. In small doses, this is a useful affect for heart problems; in higher doses, the effect is extremely toxic.

The binding of substances to the surfaces of cells produces a variety of important consequences:

1. Cardiac glycosides interfere with the energy system that drives the membrane's active ion-transport system;
2. Glycoside-coated infectious organisms, such as mycoplasma, and glycoprotein antigens, such as CEA, cause cells to clump together;
3. Protein lectins and glycoside saponins, such as ouabain, affect cell-cell recognition and even induce cell division;
4. Antibodies initiate the pinocytosis of the membrane components by some as yet unknown mechanism that is speculated to be of crucial importance in processes of cell differentiation;
5. Mediators initiate the activation of the adenylate cyclase-cAMP sequence of enzyme activation;
6. Even glycosaminoglycans (e.g., from the intercellular matrix), by their natural glycosidic affinity for various compositions of the glycocalyx, must produce important effects on cell functions, if only by the changes they cause — as, clearly, do all of the other agents just mentioned — in the electrochemical microenvironment of the cell's sole communicative link with its exterior environment, the plasma-lemma.

From the foregoing, a picture of how the eustatically effective botanical glycosides perform their functions can now be drawn. It is worthy of note at the outset that the particular glycosides in question are saponins, a group of carbohydrates that have demonstrated their affinity for cell membranes without question in their actions as cardiac glycosides. It is clear, however, that there are important distinctions between the eustatic saponins and their cardiac-affecting relatives; the plant saponin glycosides that have shown unequivocal eustatic effects have also shown — unlike the cardiac saponins — extremely low toxicity or none at all. One property the two kinds of saponins clearly do share, however, is their affinity for cell surfaces, and this is presumably how the eustatic effects are initiated. Several of these effects seem likely.

A primary and ubiquitous effect of membrane-bound eustatic glycosides, of course, would be to modify the electrochemical microenvironment of the cell membrane, perhaps leading to subtle alterations in its structure and function and, hence, the facilitation of its communicational — and homeostatic — activity.

Second, eustatic glycosides binding to the plasma-lemma may activate the adenylate cyclase chain of reactions leading to the activation of pre-formed but inactive enzymes inside the cell. These enzymes can act inside the cell or be excreted into the intercellular matrix.

7

**0 066 283**

The third major effect of plasmalemma-bound eustatic glycosides may be the triggering of endocytotic activity (pinocytosis and phagocytosis). This would carry the glycosides into the cell where their presence could initiate both the induction of genetic neosynthesis of glycosidases of various sorts and the activation of latent glycosidases liberated by lysosomes. Again, these enzymes could act both within the cell and, after excretion, in the intercellular matrix.

Outside the cell, glycosidases could perform a debridement of the glycocalyx, clearing receptor sites of occluding glycosides for enhanced access by mediators. Other glycosidases — glucosaminidases, lysozyme and hyaluronidase — could depolymerize glycosaminoglycans in the matrix, loosening the intercellular substance and increasing thereby the flow to the plasmalemma of nutrients, water and informational substances.

The latter action would, of course, generate hyaluronic acid gel-cells. These might bind to the glycocalyx and, themselves, trigger pinocytosis, thereby gaining entry to the cell, contents and all. Or other influences may initiate generalized endocytosis, producing the same effects.

Whatever the mechanism or mechanisms, it is clear that the result is a dynamic flux of cellular activity surrounding the binding and movement of glycosides and glycosaminoglycans onto and across cell boundaries, with all of the attendant consequences. The oscillations of these radiating phenomena would be miniscule — virtually undetectable, because they would take place on the level of only a few molecules (of glycosides, glycosaminoglycans, metal ion-complexes, nutrients and enzymes) at a time. Nevertheless, even these slight effects would be calculated to result in a significant shift of the homeostatic balance toward optimal physiological conditions in the organism.

Several kinds of eustatic glycosides have been identified. The most potent, least toxic and broadest ranging in effects are six saponin panaxosides and nine saponin eleutherosides, all derived from the *Araliaceae* family of plants of which *Eleutherococcus senticosus* and *Panax ginseng* are members.

Reducing agents

The eustatic function of these substances seems to be limited initially to their known ability to depolymerize intercellular hyaluronic acid, but expands to include all of the consequent effect of that action, as described in the section above on glycosaminoglycans.

Reducing eustatants include vitamin C (ascorbic acid), the amino acid cysteine, dihydroxymaleate, *i.e.*, dihydroxymaleic acid, the inactive form of the papaya enzyme papain, and other possessors of reducing thiol groups.

Chemical sources of molecular oxygen

Both molecular oxygen and divalent metal cations are required for the depolymerization of hyaluronic acid in the intercellular matrix by various reducing substances, such as those mentioned above. The effect of these adjunctive eustatants would thus be essentially the same as that of reducing agents. It should be noted, however, that while their eustatic action is properly described as adjunctive, for all practical purposes chemical sources of molecular oxygen — indeed, molecular oxygen, itself — can be expected to act, seemingly independently, as true eustatants because of the constant presence in tissues of endogenous reducing agents and metal ions that coordinate with molecular oxygen for their eustatic action.

Hydrogen peroxide is the most obvious, least toxic and cheapest example of a chemical source of molecular oxygen (actually, it is a source of atomic oxygen, but two such units combine readily in tissue into the molecular form). Other sources are NAD (nicotinamide adenine dinucleotide) which is reduced in tissues with water to $1/2O_2 + NADH$, NADP (nicotinamide dinucleotide phosphate) and superoxide dismutase.

Mild proteases

In addition to the role cited above for papain as a thiol-bearing reducing agent, proteases participate in eustatic processes much as glycosidases do, by splitting complexes that glycosaminoglycans and glycosides make with proteins, setting in train the cascade of reactions initiated by those carbohydrates in the free, depolymerized state.

Mild proteases for which eustatic-type of action has already been documented are the pineapple enzyme, bromelain, and the papaya enzyme, papain — in both active and inactive forms.

Combining eustatic compositions

Certain of the eustatic compositions disclosed herein will have cooperative, synergistic effects when combined. In the discussion of glycosaminoglycans, it was noted that hyaluronic acid gel-cells serve to carry metal ions to the genome which bind to the DNA and produce the reversible unwinding of the genome previously discussed. Exogenous administration of glycosaminoglycans, particularly hyaluronic acid, and metal ions will result in a greater incidence of gel-cell encapsulated metal ions passing through the cell membrane, contributing to a greater permeability of the intercellular substance and a greater occurrence of the reversible unwinding of the genome induced by metal ions.

It was also noted in the discussion of chemical sources of molecular oxygen that both molecular oxygen and metal cations are required for the depolymerization of hyaluronic acid in the intercellular

8

matrix by reducing agents such as ascorbid acid. A greater concentration of all of these eustatants would produce the beneficial loosening of the intercellular substance. Moreover, the addition of glycosaminoglycans, such as hyaluronic acid should further amplify the loosening effect, as well as the carriage of metal ions to the genome.

Formulations of eustatic compositions

Representatives of each of the categories of eustatic compositions discussed above can be rationally formulated — with the addition of certain supportive agents — into at least three general classes of preparations for use as pharmacological and/or nutritional, therapeutic, prophylactic and tonic preparations.

The general formulation is herein designated "EU-PAN," suggesting its capacity to promote eustatis (EU-) over a very wide range (-PAN) of physiological states and conditions. The eustatic formulations whose carbohydrate component consists principally of hyaluronic acid, obtainable from animal sources such as deproteinated cartilage, the vitreous body of the eye, the umbilical cord and the placenta, is generally designated, EU-PAN A, and is intended for use primarily as a pharmaceutical and secondarily as a nutritional supplement. The parallel formulation whose carbohydrate component consists of the non-toxic saponin glycosides derived from *Eleutherococcus Senticossus* (Siberian ginsent) or *Panax ginseng*, is generally designated "EU-PAN B," and is intended to be applied primarily as a nutritional supplement although it may also be used as a pharmaceutical.

A third general formulation, "EU-PAN C," is similar to EU-PAN A in that it involves the use of hyaluronic acid glycoside components as the carbohydrate ingredient — in this instance, a mixture of N-acetyl D-glucosamine and D-glucuronic acid. EU-PAN C may also be applied as a pharmaceutical, but is primarily intended for use as a nutritional supplement.

Following are examples of formulations of the three forms of EU-PAN with acceptable ranges and preferred amounts of constituents indicated for daily administration to a human being. These examples, and the suggested forms of administration which follow, are not intended to be limiting.

Example 1 (EU-PAN A):

|  | Range | Preferred |
|---|---|---|
| 1. Hyaluronic acid | (500 mg—10 g) | Any amount within range |
| 2. Polyvalent metal cations | | |
| a. zinc | (15 mg—30 mg) | 30 mg |
| b. copper (cupric) | (1 mg—2 mg) | 2 mg |
| 3. Ascorbic acid | (500 mg—3 g) | 2 g |
| 4. Bromelain | (50 mg—150 mg) | 100 mg |
| 5. Supportive Base | | |
| a. ribonucleic acid | (200 mg—600 mg) | 400 mg |
| b. inosital | (100 mg—600 mg) | 250 mg |
| c. pyridoxine | (100 mg—500 mg) | 200 mg |
| d. vitamin B complex | (standard) | — |
| e. dolomite (calcium and magnesium) | (standard) | — |
| f. superoxide dismutase (SOD) | (10 μg—100 μg) | 50 μg |
| g. nicotinamide | (500 mg—3 g) | 2 g |

In the preferred formula for EU-PAN A above, the constituent amount of hyaluronic acid may be obtained in almost pure form from animal sources such as the vitreous body of the eye, the umbilical cord and the placenta. The other constituents are commercially available. The above is a dry mixture suitable for administration as such, or encapsulated, or compacted into tablet form. The size and number of such capsules or pills to be conveniently administered will of course depend upon the amounts of constituents used. This example may optionally include nicotinamide adenine dinucleotide phosphate (NADP) as a

9

chemical source of molecular oxygen and the polyvalent metal cations manganese, iron (ferrous) and chromium in the following amounts:

|  | Range | Preferred |
|---|---|---|
| a. manganese | (4 mg—30 mg) | 10 mg |
| b. iron (ferrous) | (15 mg—30 mg) | 30 mg |
| c. chromium | (100 mg—200 µg) | 200 µg |

Example 2 (EU-PAN A):

|  | Range | Preferred |
|---|---|---|
| 1. Hyaluronic acid and chondroitin sulfates A and C | (500 mg—10 g) | Any amount within range |
| 2. Metal ions | (Same as Example 1) | |
| 3. Ascorbic acid | (Same as Example 1) | |
| 4. Bromelain or Papain | (30 mg—100 mg) | Any amount within range |
| 5. Supportive base | (Same as Example 1) | |

In this example of EU-PAN A, the constituent amount of hyaluronic acid and sulfated chondroitins may be obtained from deproteinated cartilage. The optional ingredients are the same as for Example 1.

Example 3 (EU-PAN A):

|  | Range | Preferred |
|---|---|---|
| 1. Hyaluronic acid or | (Same as Example 1) | |
| Hyaluronic acid and chondroitin sulfates A and C | (Same as Example 2) | |
| 2. Metal ions | (Same as Example 1) | |
| 3. Ascorbic acid | (Same as Example 1) | |
| 4. Bromelain or | (Same as Example 1) | |
| Papain | (Same as Example 1) | |
| 5. Supportive base | (Same as Example 1) | |
| 6. N-methyl-2-pyrrolidinone | — | — |

This example is the preferred liquid form of EU-PAN A which benefits from the addition of the pseudoaqueous eustatant N-methyl pyrrolidinone. Alternatives to methyl pyrrolidinone would include ethylene carbonate or hydrogen peroxide.

**0 066 283**

Example 4 (EU-PAN B):

|  | Range | Preferred |
|---|---|---|
| 1. Eleutherosides | (500 mg—10 g) | 2 g |
| 2. Polyvalent metal cations | (Same as Example 1) | |
| 3. Ascorbic acid | (Same as Example 1) | |
| 4. Bromelain | (Same as Example 1) | |
| 5. Supportive Base | (Same as Example 1) | |

In the above example, the constituent amount of eleutherosides may be obtained from Siberian ginseng *(Eleutherococcus senticossus)*. This composition may optionally include the polyvalent metal cations manganese, iron (ferrous) and chromium in the following amounts.

|  | Range | Preferred |
|---|---|---|
| a. manganese | (4 mg—30 mg) | 10 mg |
| b. iron (ferrous) | (15 mg—30 mg) | 30 mg |
| c. chromium | (100 µg—200 µg) | 200 µg |

Example 4, is a dry mixture, suitable for administration as such, or encapsulated, or compacted into tablet form.

Example 5 (EU-PAN B):

|  | Range | Preferred |
|---|---|---|
| 1. Panaxosides | (500 mg—10 g) | 2 g |
| 2. Polyvalent metal cations | (Same as Example 1) | |
| 3. Ascorbic acid | (Same as Example 1) | |
| 4. Bromelain | (Same as Example 1) | |
| 5. Supportive Base | (Same as Example 1) | |

The constituent amount of panaxosides in this example may be derived from Panax ginseng. The optional ingredients are the same as in Example 4.

Example 6 (EU-PAN B):

|  | Range | Preferred |
|---|---|---|
| 1. Eleutherosides | (Same as Example 4) | |
|     or | | |
|     Panaxosides | (Same as Example 5) | |
| 2. Polyvalent metal cations | (Same as Example 1) | |
| 3. Ascorbic acid | (Same as Example 1) | |
| 4. Bromelain | (Same as Example 1) | |
| 5. Supportive Base | (Same as Example 1) | |
| 6. Water | — | — |

The preferred liquid form of EU-PAN B above employs water in any convenient amount as the pseudoaqueous component.

11

Example 7 (EU-PAN C):

|  | Range | Preferred |
|---|---|---|
| 1. N-acetyl D-glucosamine and D-glucuronic acid | (500 mg—10 g) | Any amount within range |
| 2. Polyvalent metal cations | | (Same as Example 1) |
| 3. Ascorbic acid | | (Same as Example 1) |
| 4. Bromelain | | (Same as Example 1) |
| 5. Supportive Base | | (Same as Example 1) |

The glycosaminoglycans of EU-PAN C, namely, N-acetyl-D-glucosamine and D-glucuronic acid, may be obtained in relatively pure form from chemical suppliers. Optional ingredients are the same as for Example 1. The above is a dry mixture suitable for administration as such, or encapsulated, or compacted into tablet form. The preferred liquid form of EU-PAN C, like that of EU-PAN A, employs N-methyl-2-pyrrolidinone as its pseudoaqueous eustant.

Supportive base

While not themselves eustatic agents, the items listed in the "Supportive Base" component of the three kinds of EU-PAN formulations do, as their designation suggests, have the capacity to contribute to eustatic processes by working in concert with eustatants.

Ribonucleic acid, for example, is synthesized — and its nucleotide components thus consumed — in eustatic activity that promotes the synthesis of protein enzymes and hormones. Supplementing eustatant preparations with an RNA "digest" thus creates a ready surplus of ribonucleotides over endogenous sources. (Amino acid supplementation might similarly be justified to account for elevated levels of protein synthesis in eustasis, but this is probably provided for adequately by the normal diet.)

Inositol is suggested because of the connection this vitamin B complex substance has with glycoside activity (notably in the synthesis of growth-promoting factors in plants); pyridoxine (vitamin B-6) is associated with zinc utilization; nicotinamide (vitamin B-3) would augment endogenous reserves to promote the synthesis of nicotinamide adenine dinucleotide (a potential route to molecular oxygen in the tissues). Vitamin B complex, which is generally defined as a group of factors present in yeast, liver and whole grains, soluble in water, including thiamin hydrochloride, riboflavin, nicotinic acid, pantothenic acid, pyridoxine hydrochloride, biotin, inositol, para-amino benzoic acid, adenylic acid, choline, folic acid, and possibly vitamin B, vitamin B-4, vitamin I, vitamin J, vitamin L, vitamin M, vitamin U, vitamin W or B-W (H. Bennett, ed., *Concise Chemical and Technical Dictionary* (Chemical Publishing Co., Inc., 1974), would support the utilization of these B vitamins.

Dolomite, on the other hand, might be argued to act as a eustatant, itself, serving as it does as a convenient supplemental source of the polyvalent cations of calcium and magnesium. Those ions may, in fact, contribute to eustatic activity as other divalent cations do. However, a better established rational for including dolomite as a eustatant-supportive substance is that the divalent calcium ion is a key factor in membrane function, particularly as it pertains to information transfer in cellular communications.

Administrations

As wide a variety of administratable forms of the three EU-PANS exists as there are possible routes of administration. All should produce effective results, inasmuch as eustatants act systemically and do not select target specific organs or tissues. At the same time, certain forms of the EU-PANS should produce better results than others because of a more direct (and concentrated) exposure of a particular affected part of the organism requiring regulatory maintenance of one sort or another. Thus, a pharmacologic EU-PAN (type A, B or C) administered in the form of a suppository would be useful in a rectal conditions (such as hemorrhoids), droplet EU-PAN in eye, ear and nose conditions, a cream or ointment EU-PAN in cases of epidermal erruptions and trauma (cuts, burns, bruises), etc. The fluid form of EU-PAN should be a particular effective means of administering the eustatant formulation because the pseudoaqueous component would not only itself promote eustasis systemically but would serve, also, as a carrier of the mixture rapidly throughout the entire organism (as DMSO has shown the capacity to do). The above forms of administration can be used to improve epidermal conditions, and lead to healthier skin, hair and nails.

The nutritional EU-PAN (A, B or C) could likewise, be taken in capsule, tablet or liquid form preferably, in a flavored (non citric) beverage of some sort — or fabricated into a food product, such as a nutrient bar.

**Claims**

1. A formulation of eustatants comprising:

12

a non-toxic quantity of a polyvalent metal ion comprising a member selected from divalent zinc, manganese, the ferrous ion of iron, the cupric ion of copper and trivalent chromium; and

a glycosaminoglycan comprising a member selected from hyaluronic acid, a mixture of hyaluronic acid and chondroitin sulfates A and C, and a mixture of the components of hyaluronic acid, namely, N-acetyl glucosamine and D-glucuronic acid.

2. A formulation of eustatants comprising:

a non-toxic quantity of a polyvalent metal ion comprising a member selected from divalent zinc, manganese, the ferrous ion of iron, the cupric ion of copper and trivalent chromium; and

a non-toxic saponin glycoside comprising a member selected from the panaxosides present in the plant *Panax ginseng* and the eleutherosides present in the plant *Eleutherococcus senticosus*.

3. A eustatic formulation comprising either:

(I) EU-PAN A, comprising

(a) a glycosaminoglycan comprising a member selected from hyaluronic acid and a mixture of hyaluronic acid and chondroitin sulfates A and C,

(b) a polyvalent metal ion in an amount suitable for daily administration to an adult human, said polyvalent metal ion comprising a member selected from divalent zinc, manganese, the ferrous ion of iron, the cupric ion of copper and trivalent chromium;

(c) a reducing agent comprising a member selected from ascorbic acid, cysteine and dihydroxymaleate; and

(d) a mild protease comprising a member selected from bromelain and papain.

(II) EU-PAN B, comprising

(a) a non-toxic saponin glycoside comprising a member selected from eleutherosides and panaxosides;

(b) a polyvalent metal ion in an amount suitable for daily administration to an adult human, said polyvalent metal ion comprising a member selected from divalent zinc, manganese, the ferrous ion of iron, the cupric ion of copper and trivalent chromium, and

(c) a reducing agent consisting of ascorbic acid; or

(III) EUPAN C, comprising

(a) a glycosaminoglycan consisting of a mixture of N-acetyl D-glucosamine and D-glucuronic acid;

(b) a polyvalent metal ion in an amount suitable for daily administration to an adult human, said polyvalent metal ion comprising a member selected from divalent zinc, manganese, the ferrous ion of iron, the cupric ion of copper and trivalent chromium;

(c) a reducing agent comprising a member selected from ascorbic acid, cysteine and dihydroxymaleate; and

(d) a mild protease comprising a member selected from bromelain and papain.

4. The formulation of claim 3 wherein:

EU-PAN A further comprises

(e) a supportive base comprising a member selected from ribonucleic acid (RNA), inositol, pyridoxine, vitamin B complex, dolomite and nicotinamide;

EU-PAN B further comprises

(d) a mild protease consisting of bromelain, and

(e) a supportive base comprising a member selected from ribonucleic acid (RNA), inositol, pyridoxine, vitamin B complex, dolomite and nicotinamide; and

EU-PAN C further comprises

(e) a supportive base comprising a member selected from ribonucleic acid (RNA), inositol, pyridoxine, vitamin B complex, dolomite and nicotinamide.

5. The formulation of claim 4 wherein:

EU-PAN A further comprises

(f) a non-toxic chemical source of molecular oxygen comprising a member selected from hydrogen peroxide, nicotinamide adenine dinucleotide phosphate and superoxide dismutase;

the supportive base of EU-PAN B is ribonucleic acid (RNA), and EU-PAN B further comprises

(f) a non-toxic chemical source of molecular oxygen comprising superoxide dismutase; and

EU-PAN C further comprises

(f) a non-toxic chemical source of molecular oxygen comprising a member selected from hydrogen peroxide, nicotinamide adenine dinucleotide phosphate and superoxide dismutase.

6. The formulation of claim 5 wherein:

13

the glycosaminoglycan selected for EU-PAN A is provided in an amount within the range of 500 mg. to 10 g.;

the non-saponin glycoside selected for EU-PAN B is provided in an amount within the range of 500 mg. to 10 g.;

the mixture of N-acetyl D-glucosamine and D-glucuronic acid of EU-PAN C is provided in an amount within the range of 500 mg. to 10 g;

the polyvalent metal ion selected for EU-PAN A, or EU-PAN B or EU-PAN C is zinc provided in an amount within the range of 15 mg. to 30 mg. and copper (cupric) provided in an amount within the range of 1 mg. to 2 mg.;

the reducing agent selected for EU-PAN A, or EU-PAN B or EU-PAN C is ascorbic acid provided in an amount within the range of 500 mg. to 3 g.; and

the mild protease selected for EU-PAN A, or EU-PAN B or EU-PAN C is bromelain provided in an amount within the range of 50 mg. to 150 mg.

7. The formulation of claim 3 wherein:

the components (a)—(d) of EU-PAN A or EU-PAN C are mixed with a pseudoaqueous substance comprising one or more members selected from N-methyl-2-pyrrolidinone and ethylene cyclic carbonate.

**Patentansprüche**

1. Eustatisches Mittel enthaltend:

— eine nicht-toxische Menge eines mehrwertigen Metallions, ausgewählt aus zweiwertigem Zink, zweiwertigem Mangan, dem Eisen-II-ion, dem Kupfer-II-ion und dreiwertigem Chrom und
— ein Glykosaminoglycan, ausgewählt aus Hyaluronsäure, einem Gemisch von Hyaluronsäure und Chondroitinsulfaten A und C und einem Gemisch der Bestandteile von Hyaluronsäure, nämlich N-Acetylglucosamin und D-Glucuronsäure.

2. Eustatisches Mittel enthaltend:

— eine nicht-toxische Menge eines mehrwertigen Metallions, ausgewählt aus zweiwertigem Zink, zweiwertigem Mangan, dem Eisen-II-ion, dem Kupfer-II-ion und dreiwertigem Chrom und
— ein nicht-toxisches Saponinglycosid, ausgewählt aus den in der Pflanze Panax ginseng vorhandenen Panoxiden und den in der Pflanze Eleutherococcus senticosus vorhandenen Eleutherosiden.

3. Eustatisches Mittel enthaltend entweder:

(I) EU-PAN A, enthaltend
(a) ein Glycosaminglycan, ausgewählt aus Hyaluronsäure und einem Gemisch von Hyaluronsäure und Chondroitinsulfaten A und C,
(b) ein mehrwertiges Metallion in einer Menge, die zur täglichen Verabreichung an einen erwachsenen Menschen geeignet ist, wobei das mehrwertige Metallion ausgewählt ist aus zweiwertigem Zink, zweiwertigem Mangan, dem Eisen-II-ion, dem Kupfer-II-ion und dreiwertigem Chrom,
(c) ein Reduktionsmittel, ausgewählt aus Ascorbinsäure, Cystein und Dihydroxymaleat, und
(d) eine milde Protease, ausgewählt aus Bromelain und Papain;

(II) EU-PAN B, enthaltend:
(a) ein nicht-toxisches Saponinglykosid, ausgewählt aus Eleutherosiden und Panaxosiden,
(b) ein mehrwertiges Metallion in einer Menge, die zur täglichen Verabreichung an einen erwachsenen Menschen geeignet ist, wobei das mehrwertige Metallion ausgewählt ist aus zweiwertigem Zink, zweiwertigem Mangan, dem Eisen-II-ion, dem Kupfer-II-ion und dreiwertigem Chrom, und
(c) ein Reduktionsmittel, bestehend aus Ascorbinsäure; oder

(III) EU-PAN C, enthaltend:
(a) ein Glycosaminoglycan, bestehend aus einem Gemisch aus N-Acetyl-D-glucosamin und D-Glucuronsäure,
(b) ein mehrwertiges Metallion in einer Menge, die zur täglichen Verabreichung an einen erwachsenen Menschen geeignet ist, wobei das mehrwertige Metallion ausgewählt aus zweiwertigem Zink, zweiwertigem Mangan, dem Eisen-II-ion, dem Kupfer-II-ion und dreiwertigem Chrom,
(c) ein Reduktionsmittel, ausgewählt aus Ascorbinsäure, Cystein und Dihydroxymaleat, und
(d) eine milde Protease, ausgewählt aus Bromelain und Papain.
4. Mittel nach Anspruch 3, wobei

EU-PAN A ferner enthält:
(e) eine unterstützende Base, ausgewählt aus Ribonucleinsäure (RNS), Inosit, Pyridoxin, Vitamin-B-Komplex, Dolomit und Niotinsäureamid,

EU-PAN B ferner enthält:

(d) eine milde Protease, bestehend aus Bromelain und

(e) eine unterstützende Base, ausgewählt aus Ribonucleinsäure (RNS), Inosit, Pyridoxin, Vitamin-B-Komplex, Dolomit und Nikotinsäureamid und

EU-PAN C ferner enthält:

(e) eine unterstützende Base, ausgewählt aus Ribonucleinsäure (RNS), Inosit, Pyridoxin, Vitamin-B-Komplex, Dolomit und Nicotinsäureamid.

5. Mittel nach Anspruch 4, wobei

EU-PAN A ferner enthält:

(f) eine nicht-toxische chemische Quelle für molekularen Sauerstoff, ausgewählt aus Wasserstoffperoxid, Nicotinsäureamid-adinin-dinucleotidphosphat und Superoxid-dismutase,
die unterstützende Base von EU-PAN B Ribonucleinsäure (RNS) ist und

EU-PAN B weiterhin enthält:

(f) eine nicht-toxische chemische Quelle für molekularen Sauerstoff, umfassend Superoxid-dismutase, und

EU-PAN C ferner enthält:

(f) eine nicht-toxische chemische Quelle für molekularen Sauerstoff, ausgewählt aus Wasserstoffperoxid, Nicotinsäureamid-adinin-dinucleotidphosphat und Superoxid-dismutase.

6. Mittel nach Anspruch 5, wobei

— das für EU-PAN A ausgewählte Glycosaminoglycan in einer Menge im Bereich von 500 mg bis 10 g vorgesehen ist,
— das für EU-PAN B ausgewählte Nicht-Saponinglycosid in einer Menge im Bereich von 500 mg bis 10 g vorgesehen ist,
— das Gemisch aus N-Acetyl-D-glucosamin und D-Glucuronsäure von EU-PAN C in einer Menge im Bereich von 500 mg bis 10 g vorgesehen ist,
— das für EU-PAN A oder EU-PAN B oder EU-PAN C ausgewählte mehrwertige Metallion Zink in einer Menge im Bereich von 15 mg bis 30 mg vorgesehen ist und Kupfer-(II) in einer Menge im Bereich von 1 mg bis 2 mg vorgesehen ist,
— das für EU-PAN A oder EU-PAN B oder EU-PAN C ausgewählte Reduktionsmittel Ascorbinsäure in einer Menge im Bereich von 500 mg bis 3 g vorgesehen ist, und
— die für EU-PAN A oder EU-PAN B oder EU-PAN C ausgewählte milde Protease Bromelain in einer Menge im Bereich von 50 mg bis 150 mg vorgesehen ist.

7. Mittel nach Anspruch 3, wobei die Komponenten (a) bis (d) von EU-PAN A oder EU-PAN C mit einer pseudowässrigen Substanz, umfassend 1 oder mehrere Bestandteile, ausgewählt aus N-Methyl-2-pyrrolidinon und cyclischem Ethylencarbonat vermischt werden.

**Revendications**

1. Composition eustatique comprenant:
une quantité non toxique d'un ion métallique polyvalent comprenant un membre choisi parmi le zinc divalent, le manganèse (divalent), le ion ferreux du fer, le ion cuprique du cuivre et le chrome trivalent; et
un glycosaminoglycan comprenant un membre choisi parmi l'acide hyaluronique les sulfates A et C de chondroïtine et un mélange des components de l'acide hyaluronique, a savoir la glucosamine N-acétylée et l'acide D-glucuronique.

2. Composition eustatique comprenant:
une quantité non toxique d'un ion métallique polyvalent comprenant un membre choisi parmi le zinc divalent, le manganèse (divalent), le ion ferreux du fer, le ion cuprique du cuivre et le chrome trivalavent; et
un glycoside d'une saponine non-toxique comprenant un membre choisi parmi les panaxosides présents dans les plantes *Panax ginseng* et les éleuthérosides présents dans les plantes *Eleuthérococcus senticosus*.

3. Composition eustatique comprenant soit:

I EU-PAN A comprenant soit

(a) un glycosaminoglycan comprenant un membre choisi parmi l'acide hyaluronique et un mélange de l'acide hyaluronique et des sulfates A et C de chondroïtine,

(b) un ion métallique polyvalent en une quantité propre à être administrée à un sujet adulte, ledit ion métallique polyvalent comprenant un membre choisi parmi le zinc divalent, le manganèse (divalent), le ion ferreux du fer, le ion cuprique du cuivre et le chrome trivalent,

(c) un agent reducteur comprenant un membre choisi parmi l'acide ascorbique, la cysteïne et (un) maléate dihydroxylé, et

(d) une protéase douce comprenant un membre choisi parmi la bromélinase et la papaïne; soit

II EU-PAN B comprenant

(a) un glycoside d'une saponine non-toxique comprenant un membre choisi parmi des éléuthérosides et panaxosides,

(b) un ion métallique polyvalent en une quantité propre à être administrée à un sujet adulte, ledit ion métallique polyvalent comprenant un membre choisi parmi le zinc divalent, le manganèse (divalent), le ion ferreux du fer, le ion cuprique du cuivre et le chrome trivalent,

(c) un agent reducteur consistant en l'acide ascorbique; soit

III EU-PAN C comprenant

(a) un glycosaminoglycan consistant en un mélange de la D-glycosamine N-acetylée et l'acide glucuronique,

(b) un ion métallique polyvalent en une quantité propre à être administrée à un sujet adulte, ledit ion métallique polyvalent comprenant un membre choisi parmi le zinc divalent, le manganèse (divalent), le ion ferreux du fer, le ion cuprique du cuivre et le chrome trivalent,

(c) un agent reducteur comprenant un membre choisi parmi l'acide ascorbique, la cysteïne et (un) maléate dihydroxylé, et

(d) une protéase douce comprenant un membre choisi parmi la bromélinase et la papaïne.

4. Composition selon la revendication 3 caractérisée en ce que

EU-PAN A comprend également

(e) une base supportante comprenant un membre choisi parmi l'acide ribonucleique (ARN), l'inositol, la pyridoxine, le complex vitamine B, la dolomie et la nicotinamide,

EU-PAN B comprend également

(d) une protéase douce consistant en bromélinase, et

(e) une base supportante comprenant un membre choisi parmi l'acide ribonucleique (ARN), l'inositol, la pyridoxine, le complex vitamine B, la dolomie et la nicotinamide, et

EU-PAN C comprend également

(e) une base supportante comprenant un membre choisi parmi l'acide ribonucleique (ARN), l'inositol, la pyridoxine, le complex vitamine B, la dolomie et la nicotinamide.

5. Composition selon la revendication 4 caractérisée en ce que

EU-PAN A comprend également

(f) une source chimique non-toxique d'oxygène moléculaire comprenant un membre choisi parmi l'eau oxygénée, le phosphate du nicotinamide adenin dinucléotide et la dismutase superoxide, la base supportante d'EU-PAN B est l'acide ribonucleique et qu'EU-PAN B comprend également

(f) une source chimique non-toxique d'oxygène moléculaire comprenant de la dismutase superoxide, et en ce qu'EU-PAN C comprend également

(f) une source chimique non-toxique d'oxygène moléculaire comprenant un membre choisi parmi l'eau oxygénée, le phosphate du nicotinamide adenin dinucléotide et la dismutase superoxide.

6. Composition selon la revendication 5, caractérisée en ce que:

le glycosaminoglycan choisi pour EU-PAN A est prévu en une quantité de 500 mg à 10 g;

le glycoside non-saponine choisi pour EU-PAN B est prévu en une quantité de 500 mg à 10 g;

le mélange de D-glucosamine N-acetylée et d'acide glucuronique d'EU-PAN C est prévu en une quantité de 500 mg à 10 g;

le ion métallique polyvalent choisi pour EU-PAN A ou pour EU-PAN B ou pour EU-PAN C est du zinc prévu en une quantité de 15 mg à 30 mg, et du cuivre (cuprique) prévu en une quantité de 1 mg à 2 mg;

l'agent reducteur choisi pour EU-PAN A our pour EU-PAN B ou pour EU-PAN C est l'acide ascorbique, prévu en une quantité de 500 mg à 3 g;

la protéase douce choisie pour EU-PAN A ou EU-PAN B ou EU-PAN C est la bromélinase prévue en une quantité de 50 mg à 150 mg.

7. La composition selon la revendication 3 caractérisée en ce que:

les composés (a)—(d) d'EU-PAN A ou d'EAU-PAN C sont mélangés avec une substance pseudo-aqueuse comprenant un ou plusieurs membres choisis parmi la N-methyl-2-pyrrolidone et l'éthylene-carbonate cyclique.